# EUROPÄISCHE PATENTSCHRIFT

(11) **EP 1 374 882 B1**
(45) Veröffentlichungstag und Bekanntmachung des Hinweises auf die Patenterteilung: **01.10.2008**
(21) Anmeldenummer: 02013391.4
(22) Anmeldetag: 19.06.2002
(51) Int. Cl.: A61K 36/76, A61K 31/355, A61P 19/02

(54) **Verwendung von Wirkstoffgemischen zur Herstellung eines Medikamentes gegen rheumatische Arthritis**
Use of mixtures of active ingredients for the manufacture of a medicament for the treatment of rheumatoid arthritis
Utilisation de mixtures d'ingrédients actifs pour la manufacture d'un médicament pour le traitement de l'arthrite rhumatoide

(43) Veröffentlichungstag der Anmeldung: 02.01.2004
(73) Patentinhaber: Cognis IP Management GmbH, 40589 Düsseldorf (DE)
(72) Erfinder: Arias, Carmen, 08190 Sant Cugat del Vallés (ES); Rull Prous, Santiago, 08034 Barcelona (ES); Fabry, Bernd, Dr., 41352 Korschenbroich (DE)

(56) Entgegenhaltungen:
- EP-A- 0 279 867
- EP-A- 0 535 283
- EP-A- 0 742 012
- WO-A-00/57876
- WO-A-01/91766
- WO-A-01/91767
- WO-A-94/13265
- DE-A- 3 405 240
- DE-A- 3 800 101
- WAGNER I ET AL: "Willow bark in the treatment of rheumatic disorders: Randomized clinical trials." PHYTOMEDICINE (JENA), Bd. 7, Nr. Supplement 2, 2000, Seite 91 XP001105164 3rd International Congress on Phytomedicine;Munich, Germany; October 11-13, 2000 ISSN: 0944-7113
- DATABASE WPI Section Ch, Week 200216 Derwent Publications Ltd., London, GB; Class D13, AN 2002-117904 XP002217915 & JP 2001 314173 A (KYOTO EIYO KAGAKU KENKYUSHO KK), 13. November 2001 (2001-11-13)
- APPELBOOM T: "ARTHROPATHY IN ART AND THE HISTORY OF PAIN MANAGEMENT-THROUGH THE CENTURIES TO CYCLOOXYGENASE-2 INHIBITORS" RHEUMATOLOGY, OXFORD UNIVERSITY PRESS, LONDON, GB, Bd. 41, Nr. SUPPL 1, April 2002 (2002-04), Seiten 28-34, XP001105161 ISSN: 1462-0324

## Beschreibung

### Gebiet der Erfindung

Die Erfindung befindet sich auf dem Gebiet der Pharmazie und betrifft die Verwendung von synergistischen Mischungen aus Tocopherolen und speziellen Pflanzenextrakten bzw. deren aktiven Wirkstoffen zur Herstellung von Medikamenten gegen rheumatische Arthritis.

### Stand der Technik

Unter Rheumatismus versteht man akute oder auch chronische Erkrankungen des Bewegungsapparates. Bekanntestes Beispiel ist sicher die chronische Polyarthritis oder rheumatoide Arthritis (RA), die zu Entzündungen und Deformationen der Gelenke führt. Dabei werden, oft symmetrisch, vor allem die kleinen Finger- und Zehengelenke, aber auch große Gelenke befallen, was mit meist reißenden oder ziehenden Schmerzen, morgendlicher Steifigkeit, Schwellungen, Funktionseinschränkungen und knotenförmigen Verdickungen unter der Haut (Rheumaknoten) einhergeht. Gemeinsam sind allen rheumatischen Erkrankungen entzündliche Reaktionen, die z.T. auf genetische Veranlagung (verbunden mit HLA Genen, wie z.B. DR4) insbesondere aber auf Autoimmunprozesse zurückgeführt werden können. So finden sich vielfach Antikörper gegen Immunglobuline im Serum der Erkrankten. Nachdem epidemilogische Studien gezeigt haben, dass RA insbesondere solche Personen befällt, die einen geringen Spiegel an Antioxidantien (wie z.B. Carotine, Tocopherole, Selen etc.) aufweisen, scheinen auch reaktive Sauerstoffspezies (sogenannte "ROS") beim Entstehen dieser Krankheit eine Rolle zu spielen. RA-Patienten zeichnen sich zudem dadurch aus, dass sie einen geringen Gehalt an Vitamin C im Blut besitzen, eine geringe Erythrozyten Superoxid-Dismutase (SOD)-Aktivität aufweisen, während der Gehalt an Thiobarbitursäure-aktiven Substanzen signifikant erhöht ist.

Bislang gibt es für die rheumatische Arthritis keine zuverlässige Behandlungsmethode. Die im Markt befindlichen Antirheumatika, bei denen es sich z.B. um nicht-steroidale Antiphlogistika oder Corticosteroide handelt, sind lediglich in der Lage, die Symptome zu lindern bzw, das Ausbrechen der Krankheit zu verzögern. Auch die biochemischen Prozesse, die RA bedingen, sind derzeit noch weitgehend unbekannt. Sicher scheint lediglich zu sein, dass polymorphonucleare Neutrophile (PMNs), Monocyten und Marcrophagen an der erhöhten ROS-Aktivität beteiligt sind. Insbesondere die PMNs zeigen eine verstärkte Chemoluminiszensantwort zu fMLP. Das Hydroxylradikal ist dabei offenbar in der Lage, die Struktur der menschlichen Immunglobuline der Klasse M (IgM) zu verändern und dadurch die Produktion von Rheumafaktoren zu stimulieren.

Aus einer Studie von DeBandt et el. in Arthritis Rheumatism 46(2), p552-532 (2002) ist bekannt, dass Tocopherole, oral aufgenommen, die durch RA bedingte Deformation von Gelenken durch eine wenigstens partielle Inhibierung der Synthese von Interleukin-1 β (IL-1 β), dem wichtigsten der in der Gelenkdeformation involvierten Cytokinen, deutlich verringert. Die Wirkung der Tocopherole ist jedoch bislang nicht optimal.

Folglich hat die Aufgabe der vorliegenden Erfindung darin bestanden, Synergisten zu finden, die die Bildung von IL-1β stärker inhibieren, als dies Tocopherole alleine vermögen. Des weiteren sollten die Mittel die Bildung von freien Sauerstoffverbindungen (ROS) vermindern.

### Beschreibung der Erfindung

Gegenstand der Erfindung ist die Verwendung von Mischungen, enthaltend
(a) Tocopherole und
(b) Extrakte von Pflanzen der Gattung *Salix* bzw. deren Wirkstoffe, ausgewählt aus der Gruppe, die gebildet wird von Salicin, Salicortin und Tremulacin,
zur Herstellung eines Medikamentes gegen rheumatische Arthritis.

Überraschenderweise wurde gefunden, dass die Salix-Extrakte bzw. die darin enthaltenen wirkenden Prinzipien in Kombination mit Tocopherolen zu einer synergistischen Inhibierung der Interleukin-1 β-Synthese und damit zu einer Verminderung der durch rheumatische Arthritis bedingten Gelenkdeformationen führt. Gleichzeitig wurde gefunden, dass die Mittel auch die Bildung von freien Sauerstoffverbindungen reduzieren, so dass den Entzündungsursachen der rheumatischen Arthritis gleich in zweifacher Weise entgegengewirkt wird.

### Tocopherole

Unter dem Begriff Tocopherole sind die in 2-Stellung mit einem 4,8,12-Trimethyltridecyl-Rest substituierten Chroman-6-ole (3,4-Dihydro-2H-1-benzopyran-6-ole) zu verstehen, die auch als Biochinone bezeichnet werden. Typische Beispiele sind die Plastichinone, Tocopherolchinone, Ubichinone, Bovichinone, K-Vitamine und Menachinone (z.B. 2-Methyl-1,4-naphthochinone). Vorzugsweise handelt es sich um die Chinone aus der Vitamin-E-Reihe, d.h. α-, β-, γ-, δ- und ε-Tocopherol, wobei letzteres noch über die ursprüngliche ungesättigte prenylseitenkette verfügt (s. Abbildung).

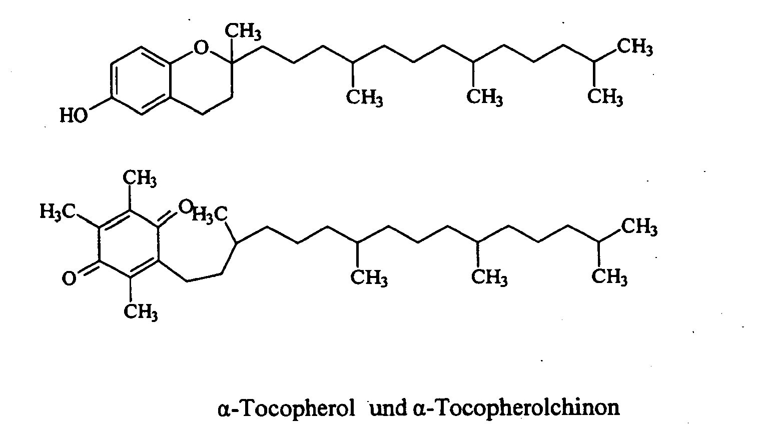

Daneben kommen auch Tocopherolchinone und -hydrochinone sowie die Ester der Chinone mit Carbonsäuren, wie z.B. Essigsäure, Bernsteinsäure, Palmitinsäure oder Phosphorsäure in Frage. Der Einsatz von α-Tocopherol, Tocopherolacetat, Tocopherolsuccinat, Tocopherolpalmitat und Tocopherolphosphat sowie deren Gemische ist bevorzugt.

### Salix

Unter der lateinischen Bezeichnung *Salix* (pharmakologisch: *Salix cortex)* verbirgt sich die Pflanzengattung der Weiden, die in ganz Europa, Asien und Nordamerika verbreitet ist. Extrakte aus Weidenrinde wurden schon im antiken Griechenland zu therapeutischen Zwecken eingesetzt, verbreitet im Mittelalter war ein Weidenaufguss als fiebersenkendes Mittel. Im Sinne der vorliegenden Erfindung sind unter Salix-Extrakten Zubereitungen zu verstehen, die beispielsweise auf Basis von *Salix alba, Salix purpurea, Salix* fragilis, *Salix pentandra* und/oder *Salix daphnoides* erhalten werden. Anstelle der Weidenextrakte können auch die aktiven Prinzipien eingesetzt werden, bei denen es sich um phenolische Glykoside und überwiegend um Salicylate, wie insbesondere Salicin (s.u.), Salicortin und Tremulacin handelt.

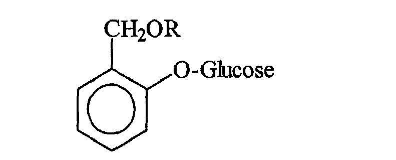

### Extraktion

Die Herstellung der Extrakte kann in an sich bekannter Weise erfolgen, d.h. beispielsweise durch wässrigen, alkoholischen oder wässrig-alkoholischen Auszug der Wurzeln. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.% aufweisen, können als solche eingesetzt werden, es ist jedoch ebenfalls möglich, das Lösungsmittel durch Trocknung, insbesondere durch Sprüh- oder Gefriertrocknung vollständig zu entfernen. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage. Vorzugsweise werden solche Extrakte eingesetzt, die einen Wirkstoffgehalt im Bereich von 5 bis 90 Gew.-% aufweisen.

In den Endzubereitungen können die Komponenten (a) und (b) in Summe in Mengen von 0,1 bis 100, vorzugsweise 1 bis 50 und insbesondere 5 bis 35 Gew.% enthalten sein, wobei man die Komponenten (a) und (b) üblicherweise im Gewichtsverhältnis 90 : 10 bis 10 : 90, vorzugsweise 75 : 25 bis 25 : 75 und insbesondere 60 : 40 bis 40 : 60 einsetzt.

### Gewerbliche Anwendbarkeit

Gegenstände der vorliegenden Erfindung betreffen die Verwendung von Mischungen, enthaltend
(a) Tocopherole und
(b) Extrakte von Pflanzen der Gattung *Salix* bzw. deren Wirkstoffe, ausgewählt aus der Gruppe, die gebildet wird von Salicin, Salicortin und Tremulacin,
zur Herstellung von Medikamenten gegen rheumatische Arthritis, wobei die Medikamente (a) die Synthese von Interleukin-1β und (b) die Freisetzung von reaktiven Sauerstoffverbindungen (ROS) inhibieren.

### Beispiele

### Inhibierung der Interleukin- 1β-Synthese

Die Inhibierung der Interleukin-Synthese wurde analog der Vorschrift in Arthritis & Rheumatism 46(2), p524 (2002) mit einem ELISA Testkit der Firma R&D Systems Europe (Abingdon, UK) untersucht. Zu diesem Zweck wurde dem Futter einer ersten Gruppe von transgenen Mäusen, die typischerweise innerhalb des ersten Lebensmonats Polyarthritis entwickeln, über einen Zeitraum von 6 Wochen 0,25 mg der Testmischungen in 100 µl Sonnenblumenöl zugemischt. Zwei weitere Gruppen erhielten über den gleichen Zeitraum als Kontrolle lediglich Sonnenblumenöl bzw. 0,25 mg Tocopherol in 100 µl Sonnenblumenöl verabreicht. Im Abstand von 1 Woche wurden Blutproben entnommen und auf die Menge an gebildetem Interleukin untersucht. Die Ergebnisse sind in Tabelle 1 zusammengefasst. Die Beispiele 1 und 2 sind erfindungsgemäß, V1 und V2 dienen zum Vergleich.

**Tabelle 1**

| **Inhibierung der Interleukin-1β-Synthese** | | | | | | | |
|---|---|---|---|---|---|---|---|
| **Bsp.** | **Testprodukt** | **Interleukin-1β=Gehalt [pg,ml] nach ...** | | | | | |
| | | **1w** | **2w** | **3w** | **4w** | **5w** | **6w** |
| V1 | Kontrolle | 40 | 60 | 120 | 100 | 90 | 80 |
| V2 | Tocopherol | 20 | 50 | 50 | 55 | 45 | 40 |
| 1 | Mischung A¹⁾ | 11 | 28 | 26 | 33 | 31 | 31 |
| 2 | Mischung B²⁾ | 12 | 31 | 31 | 33 | 33 | 31 |

| | | | | | | | |
|---|---|---|---|---|---|---|---|
| 1) Mischung aus Tocopherol / gefriergetrockneter Extrakt von Salix alba im Gewichtsverhältnis 1:1 2) Mischung aus Tocopherol / gefriergetrockneter Extrakt von Salix alba im Gewichtsverhältnis 1:2 | | | | | | | |

Die erfindungsgemäßen Mischungen inhibieren die Interleukin-Synthese deutlich stärker als das Tocopherol alleine.

### Inhibierung der Bildung reaktiver Sauerstoffverbindungen (ROS)

Rheumatische Arthritis stellt, wie oben schon erläutert, eine Entzündungserkrankung dar, welche im Zusammenhang mit der Entwicklung von reaktiven Sauerstoffverbindungen (ROS) gesehen wird. Eine Aufgabe der vorliegenden hatte daher darin bestanden, solche Mittel zur Verfügung zu stellen, die zusätzlich die Entzündungsursachen, d.h. die Bildung von ROS bekämpfen. Im Verlauf einer Entzündung werden Leucocyten, wie beispielsweise die polymorphonuclearen neutrophilen Granulocyten (PMN) durch Peptide wie etwa Cytokine stimuliert, Botschafterstoffe wie z.B. Leukotrien auszusenden, die von aktivierten oder nekrotischen Zellen in der Dermis freigesetzt werden. Diese aktivierte PMN setzen nicht nur proinflammatorische Cytokine, Leukotriene und Proteasen, sondern auch ROS, wie z.B. Superoxide und Hypochloritanionen frei. Diese Aktivität der PMN während der Inflammation ist als sogenannter Atmungsausbruch (_{"}respiratory burst") bekannt. Zur Untersuchung, inwiefern die Testsubstanzen den Atmungsausbruch verhindern oder mindern können, wurde eine Zelllinie von menschlichen leukemischen Granulocyten dieser PMN zusammen mit den Testsubstanzen bei 37 °C in Gegenwart von 5 Vol.-% CO₂ inkubiert. Nach Auslösung des Atmungsausbruchs durch Zugabe eines Hefeextraktes (Zymosan) zur Zelllösung, wurde die Freisetzung von Superoxidanionen über deren Reaktion mit Luminol bestimmt. Die Ergebnisse sind in Tabelle 2 zusammengefasst. Angegeben ist die Menge an freigesetzten ROS in %-rel zum Standard als Mittelwert einer Messreihe mit Dreifachbestimmung. Die Beispiele 3 und 4 sind erfindungsgemäß, die Beispiele V3 und V4 dienen zum Vergleich.

**Tabelle 2**

| **Anti-inflammatorische Wirkung (Angabe in %-rel.)** | | | |
|---|---|---|---|
| **Bsp.** | **Testprodukt** | **Konz. Gew.-%** | **Freigesetzte ROS** |
| V3 | Kontrolle | | 100 |
| V4 | Tocopherol | 0,05 | 99 |
| 3 | Mischung A¹⁾ | 0,05 | 88 |
| 4 | Mischung B²⁾ | 0,05 | 91 |

| | | | |
|---|---|---|---|
| 1) Mischung aus Tocopherol / gefriergetrockneter Extrakt von Salix alba im Gewichtsverhältnis 1:1 2) Mischung aus Tocopherol / gefriergetrockneter Extrakt von Salix alba im Gewichtsverhältnis 1:2 | | | |

Die Ergebnisse zeigen, dass die Testsubstanzen einen starken inhibierenden Einfluss auf den Atmungsausbruch menschlicher Granulocyten besitzen, ohne die Granulocyten zu schädigen.

## Patentansprüche

1. Verwendung von Mischungen, enthaltend
(a) Tocopherole und
(b) Extrakte von Pflanzen der Gattung *Salix*
zur Herstellung eines Medikamentes gegen rheumatische Arthritis.

2. Verwendung von Mischungen, enthaltend
(a) Tocopherole und
(b) Wirkstoffe, ausgewählt aus der Gruppe, die gebildet wird Salicin, Salicortin und Tremulacin,
zur Herstellung eines Medikamentes gegen rheumatische Arthritis.

3. Verwendung nach den Ansprüchen 1 und/oder 2, **dadurch gekennzeichnet, dass** man als Komponente (a) α-Tocopherol, Tocopherolacetat, Tocopherolsuccinat, Tocopherolpalmitat, Tocopherolphosphat sowie deren Gemische einsetzt.

4. Verwendung nach mindestens einem der Ansprüche 1 bis 3, **dadurch gekennzeichnet, dass** man Extrakte einsetzt, die einen Wirkstoffgehalt im Bereich von 5 bis 90 Gew.% einsetzt.

5. Verwendung nach mindestens einem der Ansprüche 1 bis 4, **dadurch gekennzeichnet, dass** man die Komponenten (a) und (b) im Gewichtsverhältnis 90 : 10 bis 10 : 90 einsetzt.

6. Verwendung von Mischungen nach den Ansprüchen 1 und/oder 2, wobei das Medikament die Interleukin-1β-Synthese inhibiert.

7. Verwendung von Mischungen nach den Ansprüchen 1 und/oder 2, wobei das Medikament die Freisetzung von reaktiven Sauerstoffverbindungen (ROS) inhibiert.

## Claims

1. Use of mixtures comprising
(a) tocopherols and
(b) extracts of plants of the genus Salix
for the manufacture of a medicament for rheumatoid arthritis.

2. Use of mixtures comprising
(a) tocopherols and
(b) active ingredients selected from the group which is formed salicin, salicortin and tremulacin,
for the manufacture of a medicament for rheumatoid arthritis.

3. Use according to Claims 1 and/or 2, **characterized in that** α-tocopherol, tocopherol acetate, tocopherol succinate, tocopherol palmitate, tocopherol phosphate and mixtures thereof are employed as component (a).

4. Use according to at least one of Claims 1 to 3, **characterized in that** extracts which employ an active ingredient content in the range from 5 to 90% by weight are employed.

5. Use according to at least one of Claims 1 to 4, **characterized in that** components (a) and (b) are employed in the ratio 90:10 to 10:90 by weight.

6. Use of mixtures according to Claims 1 and/or 2, where the medicament inhibits interleukin-1β synthesis.

7. Use of mixtures according to Claims 1 and/or 2, where the medicament inhibits release of reactive oxygen compounds (ROS).

## Revendications

1. Utilisation de mélanges renfermant:
(a) des tocophérols, et
(b) des extraits de plantes du genre *Salix*
pour la préparation d'un médicament pour le traitement de l'arthrite rhumatoïde

2. Utilisation de mélanges renfermant :
(a) des tocophérols, et
(b) des matières actives choisies dans le groupe qui est formé par la salicine, la salicortine et la trémulacine,
pour la préparation d'un médicament pour le traitement de l'arthrite rhumatoïde.

3. Utilisation selon les revendications 1 et/ou 2,
**caractérisée en ce qu'**
on utilise en tant que composants (a) de l'α-tocophérol, de l'acétate de tocophérol du succinate de tocophérol, du palmitate de tocophérol, du phosphate de tocophérol et leurs mélanges.

4. Utilisation selon au moins l'une des revendications 1 à 3,
**caractérisée en ce qu'**
on utilise des extraits qui présentent une teneur en matière active dans la plage de 5 % à 90 % en poids.

5. Utilisation selon au moins l'une des revendications 1 à 4,
**caractérisée en ce qu'**
on utilise les composants (a) et (b) dans une proportion pondérale de 90:10à10:90.

6. Utilisation de mélanges selon les revendications 1 et/ou 2
dans laquelle
le médicament inhibe la synthèse de l'interleukine-1β.

7. Utilisation de mélanges selon les revendications 1 et/ou 2,
dans laquelle
le médicament inhibe la libération de composés oxygénés réactifs (ROS).
